# EUROPÄISCHE PATENTSCHRIFT

(11) **EP 1 230 251 B1**
(45) Veröffentlichungstag und Bekanntmachung des Hinweises auf die Patenterteilung: **11.06.2003**
(21) Anmeldenummer: 00974414.5
(22) Anmeldetag: 18.10.2000
(51) Int. Cl.: C07H 15/04, C11D 1/66

(54) **VERFAHREN ZUR HERSTELLUNG VON FESTEN ZUCKERTENSIDEN**
METHOD FOR PRODUCING SOLID SUGAR SURFACTANTS
PROCEDE DE PRODUCTION D'AGENTS TENSIOACTIFS DE TYPE SUCRE SOLIDES

(30) Priorität: 27.10.1999 DE 19951598
(43) Veröffentlichungstag der Anmeldung: 14.08.2002
(73) Patentinhaber: Cognis Deutschland GmbH & Co. KG, 40589 Düsseldorf (DE)
(72) Erfinder: WEUTHEN, Manfred, 40764 Langenfeld (DE); SCHMID, Karl, Heinz, 40822 Mettmann (DE)
(86) Internationale Anmeldenummer: EP0010266
(87) Internationale Veröffentlichungsnummer: WO01030792

(56) Entgegenhaltungen:
- WO-A-92/13938
- WO-A-97/03165
- DE-A- 19 702 845

## Beschreibung

### Gebiet der Erfindung

Die Erfindung befindet sich auf dem Gebiet der Herstellung fester Zuckertenside und betrifft ein Extraktionsverfahren zur Herstellung von festen Alk(en)yloligoglykosiden.

### Stand der Technik

Alk(en)yloligoglykoside stellen wegen ihrer ausgezeichneten anwendungstechnischen Eigenschaften und der hohen ökotoxikologischen Verträglichkeit wichtige Basisrohstoffe beispielsweise für die Herstellung von kosmetischen Zubereitungen oder manuellen Spülmitteln dar. Üblicherweise werden diese Tenside in Form wäßriger Pasten verarbeitet, für eine Reihe von Anwendungen, beispielsweise im Bereich der Stückseifen oder Waschmittel, besteht aber auch ein besonderes Interesse an festen Zubereitungen, die sich leichter verarbeiten lassen und nicht erst von unerwünschten Wasseranteilen befreit werden müssen.

Aus dem Stand der Technik sind verschiedene Verfahren zur Herstellung solcher festen Zuckertenside bekannt. So wird beispielsweise in der internationalen Patentanmeldung **WO 97/03165** (Henkel) die gleichzeitige Trocknung und Granulierung von zeolithhaltigen Zuckertensidpasten in der Wirbelschicht beschrieben. Gegenstand der beiden intemationalen Patentanmeldungen **WO 97/10324** und **WO 98/40460** (Henkel) ist die Entwässerung von Zuckertensidpasten in einem horizontalen Dünnschichtverdampfer.

Durch den hohen technischen Aufwand sind diese Verfahren jedoch aus ökonomischen Gründen wenig interessant. Demzufolge hat die Aufgabe der vorliegenden Erfindung darin bestanden, ein vereinfachtes Verfahren zur Herstellung von festen Zuckertensiden zur Verfügung zu stellen, welches insbesondere auf die destillative Abtrennung von überschüssigem Fettalkohol verzichtet.

### Beschreibung der Erfindung

Gegenstand der Erfindung ist ein Verfahren zur Herstellung fester Zuckertenside, welches sich dadurch auszeichnet, daß man Glykosen in an sich bekannter Weise mit Fettalkoholen acetatisiert und die technischen Mischungen, welche neben den Alkyl- und/oder Alkenylglykosiden wenigstens noch Anteile an überschüssigem Fettalkohol enthalten, mit festen Extraktionsmitteln in Kontakt bringt, und dabei eine erste feste Phase, welche das Extraktionsmittel zusammen mit dem ganz überwiegenden Teil der Alkylund/oder Alkenyloligogiykosiden und einem gegenüber der Ausgangsmischung verminderten Anteil an Fettalkoholen und eine zweite flüssige Phase erzeugt, welche überwiegend aus abgetrenntem Fettalkohol besteht.

Überraschenderweise wurde gefunden, daß sich gemäß dem vorgeschlagenen Verfahren in technisch einfacher Weise und zu geringen Herstellkosten feste Zuckertenside enthalten lassen, die sich durch einen geringen Anteil an freien Fettalkoholen auszeichnen. Die dabei erhältlichen Produkte weisen in der Regel 20 bis 70 und insbesondere 30 bis 50 Gew.-% Glykoside auf.

### Alkyl- und/oder Alkenyloligoglykoside

Unter dem Begriff Alkyl- und Alkenyloligoglykoside werden im Sinne des erfindungsgemäßen Verfahrens nichtionische Tenside verstanden, die vorzugsweise der Formel **(I)** folgen,

**R**^{**1**}**O-[G]**_{**p**} (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Wenn von der Herstellung dieser Stoffe in unkritischer, d.h. in an sich bekannter Weise die Rede ist, dann sei stellvertretend für das umfangreiche Schrifttum auf die Schriften **EP-A1 0 301 298** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Unter technischen Gemischen, die dann der Extraktion unterworfen werden, sind solche Zwischenprodukte zu verstehen, die unmittelbar nach der Acetalisierung anfallen, also neben dem Zielprodukt im wesentlichen noch nicht umgesetzten Fettalkohol enthalten. Für das erfindungsgemäße Verfahren ist es dabei zwar bevorzugt, jedoch nicht erforderlich, daß der saure Katalysator vor der Extraktion durch Zugabe einer Base neutralisiert wird. Dies kann vielmehr auch durch Auswahl des Extraktionsmittels geschehen.

### Extraktionsmittel

Bei den Extraktionsmitteln empfiehlt es sich die Auswahl unter solchen zu treffen, welche gegenüber Alkyl- und/oder Alkenyloligoglykosiden eine höhere Hydrophilie als Fettalkohole und eine große Oberfläche aufweisen. Vorzugsweise werden solche Mittel eingesetzt, welche wenigstens nach Adsorption der Alkyl- und/oder Alkenyloligoglykosiden bei Raumtemperatur in festem Zustand vorliegen und insbesondere schon zu Beginn der Extraktion bei Raumtemperatur fest vorliegen. Typische Beispiele für geeignete Extraktionsmittel sind die folgenden:
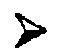 **Zeolithe**. Als Extraktionsmittel können beispielsweise die als Waschmittelbuilder häufig eingesetzten feinkristallinen, synthetischen und gebundenes Wasser enthaltende Zeolithe A und/oder P eingesetzt werden. Als Zeolith P wird beispielsweise Zeolith MAP® (Handelsprodukt der Firma Crosfield) besonders bevorzugt. Geeignet sind jedoch auch Zeolith X sowie Mischungen aus A, X und/oder P wie auch Y. Von besonderem Interesse ist auch ein cokristallisiertes Natrium/Kalium-AluminiumSilicat aus Zeolith A und Zeolith X, welches als VEGOBOND AX® (Handelsprodukt der Firma Condea Augusta S.p.A.) im Handel erhältlich ist.
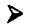 **Silicate.** Geeignete Extraktionsmittel sind weiterhin kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung **EP 0164514 A1** beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt, wobei β-Natriumdisilicat beispielsweise nach dem Verfahren erhalten werden kann, das in der internationalen Patentanmeldung **WO 91/08171** beschrieben ist. Weitere geeignete Schichtsilicate sind beispielsweise aus den Patentanmeldungen **DE 2334899 A1, EP 0026529 A1** und **DE 3526405 A1** bekannt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilicate, die zur Gruppe der mit Wasser quellfähigen Smectite zählen, sind z.B. solche der allgemeinen Formeln

   (OH)₄Si_{8-y}Al_{y}(MgₓAl₄₋ₓ)O₂₀ Montmorrilonit

   (OH)₄Si_{8-y}Al_{y}(Mg_{6-z}Li_{z})O₂₀ Hectorit

   (OH)₄Si_{8-y}Al_{y}(Mg_{6-z}Al_{z})O₂₀ Saponit

   mit x = 0 bis 4, y = 0 bis 2, z = 0 bis 6. Zusätzlich kann in das Kristallgitter der Schichtsilicate gemäß den vorstehenden Formeln geringe Mengen an Eisen eingebaut sein. Femer können die Schichtsilicate aufgrund ihrer ionenaustauschenden Eigenschaften Wasserstoff-, Alkali-, Erdalkaliionen, insbesondere Na⁺ und Ca²⁺ enthalten. Die Hydratwassermenge liegt meist im Bereich von 8 bis 20 Gew.-% und ist vom Quellzustand bzw. von der Art der Bearbeitung abhängig. Brauchbare Schichtsilicate sind beispielsweise aus **US 3,966,629, US 4,062,647, EP 0026529 A1** und **EP 0028432 A1** bekannt. Vorzugsweise werden Schichtsilicate verwendet, die aufgrund einer Alkalibehandlung weitgehend frei von Calciumionen und stark färbenden Eisenionen sind. Zu den bevorzugten Extraktionsmitteln gehören auch amorphe Natriumsilicate mit einem Modul Na₂O : SiO₂ von 1 : 2 bis 1 : 3,3, vorzugsweise von 1 : 2 bis 1 : 2,8 und insbesondere von 1 : 2 bis 1 : 2,6. Im Rahmen dieser Erfindung wird unter dem Begriff "amorph" auch "röntgenamorph" verstanden. Dies heißt, daß die Silicate bei Röntgenbeugungsexperimenten keine scharfen Röntgenreflexe liefern, wie sie für kristalline Substanzen typisch sind, sondem allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Extrakttonseigenschaften führen, wenn die Silicatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silicate werden beispielsweise in der deutschen Patentanmeldung DE 4400024 A1 beschrieben. Insbesondere bevorzugt sind verdichtete/kompaktierte amorphe Silicate, compoundierte amorphe Silicate und übertrocknete röntgenamorphe Silicate.
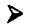 **Polymere und Polysaccharide.** Als Polymere können beispielsweise Proteinhydrolysate, Polyamide, Polyacrylate, Polyurethane und Potyvinyipyrrolidone eingesetzt werden. Auch Harnstoff und Polyhamstoff sind für diesen Zweck geeignet. Als Polysaccharide kommen beispielsweise Cellulosen, Cellutosederivate, Stärken oder Stärkehydrolysate in Frage.
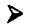 **Salze anorganischer Säuren.** Typische Beispiele sind die Alkali- und/oder Erdalkalisalze, Aluminium- oder Zinksalze der Salzsäure, Schwefelsäure, Salpetersäure, Phosphorsäure, Borsäure und Kieselsäure, wobei insbesondere die Alkalicarbonate, Alkalihydrogencarbonate, Alkalisulfate, Alkaliborate und -perborate, die verschiedenen Alkalisilicate ("Wassergläser") und Alkaliphosphate genannt werden sollen. Typische Beispiele sind Magnesiumsulfat-Heptahydrat oder Borax.
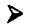 **Salze organischer Säuren.** Typische Beispiele sind die Alkali- und/oder Erdalkalisalze, Aluminiumoder Zinksalze von Monocarbonsäuren mit 1 bis 22 Kohlenstoffatomen, als da sind Ameisensäure, Essigsäure, Propionsäure, Buttersäure, Valeriansäure, Capronsäure, Caprylsäure, 2-Ethylhexansäure. Besonders bevorzugt ist der Einsatz von Natriumacetat. Anstelle der Monocarbonsäuren können auch entsprechende C₂-C₆-Dicarbonsäuren eingesetzt werden, so daß als geeignete Extraktionsmittel in gleicher Weise wie oben auch die entsprechenden Salze der Bemsteinsäure, Maleinsäure, Fumarsäure, Glutarsäure und Adipinsäure in Frage kommen. Schließlich können auch Salze hydroxyfunktionalisierter mehrwertiger Carbonsäuren eingesetzt werden, wie z.B. die oben genannten Salze von Äpfelsäure, Weinsäure und insbesondere Citronensäure. Hier ist ganz besonders der Einsatz von Alkalicitraten bevorzugt. Sofern die den Salzen zugrundeliegenden organischen Säuren bei Raumtemperatur fest sind, können auch diese eingesetzt werden. Dies trifft insbesondere für die Citronensäure zu, deren Verwendung als Extraktionsmittel besonders bevorzugt ist.

Die Extraktion kann in unterschiedlicher Weise erfolgen. Es ist beispielsweise möglich, die technische Glykosidmischung mit dem Extraktionsmittel **intensiv** zu vermischen und dann den Fettalkohol durch Filtration abzutrennen. Ebenfalls möglich ist die Extraktion mit Hilfe einer Säule, welche das Extraktionsmittel enthält. Bei diesen Verfahrensvarianten stellt sich im Extrakt in der Regel ein Gewichtsverhältnis von Glykosid zu Fettalkohol von 1 : 2 bis 50 : 1, vorzugsweise 2 : 1 bis 20 : 1 und insbesondere 5 : 1 bis 10 : 1 ein. Das Gewichtsverhältnis zwischen eingesetzter Mischung und Extraktionsmittel kann dabei 5 : 1 bis 1 : 5, vorzugsweise 1 : 1 bis 2 : 1 betragen. Gemeinsam ist den verschiedenen Ausführungsformen des erfindungsgemäßen Verfahrens femer, daß man vorzugsweise die feste Phase nach der Extraktion zerkleinert und auf die gewünschte Teilchengröße vermahlt, während man den rückgewonnenen Fettalkohol, der eine sehr hohe Reinheit besitzt, wieder in die Acetalisierung zurückführt.

### Gewerbliche Anwendbarkeit

Die nach dem erfindungsgemäßen Verfahren erhältlichen Produkte eignen sich beispielsweise zur Herstellung von festen Detergentien, wie beispielsweise Pulvem, Kompaktaten, Tabletten oder Stücken, insbesondere im Bereich der Reinigung harter Oberflächen, in denen sie in Mengen von 1 bis 50, vorzugsweise 2 bis 25 und insbesondere 5 bis 15 Gew.-% - bezogen auf die Mittel - enthalten sein können.

### Beispiele

**Beispiel 1.** 1 mol (180 g) Glucose wurde mit 4,5 mol (860 g) C_{12/14}-Kokosfettalkohol in Gegenwart von p-Toluolsuifonsäure in an sich bekannter Weise acetalisiert. Das Reaktionswasser wurde bei der Kondensation kontinuierlich abgetrennt und die Reaktion abgebrochen, als der Rest-Glucosegehalt unter einen Wert von 0,5 Gew.-% abgesunken war. Das saure Umsetzungsprodukt wurde durch Zugabe von Magnesiumoxid neutralisiert, auf 60 °C abgekühlt und unter starkem Rühren mit 500 g feinkömiger Cellulose versetzt. Nach der Homogenisierung wurde die Mischung über ein Filter, welches mit weiteren 100 g Cellulose belegt war, filtriert. Nach dem Erstarren wurde der Filterkuchen vermahlen. Es wurden 980 g Produkt mit einem Gehalt von 33,7 Gew.-% Kokosalkyloligoglucosid und 15,3 Gew.-% Fettalkohol erhalten.

**Beispiel 2.** Eine Glassäule (Länge 1 m, Durchmesser 10 cm) wurde auf 50 °C erhitzt und mit einer Mischung aus Soda und C_{12/14}-Kokosfettalkohol (Gewichtsverhältnis 1:1) befüllt. Anschließend wurde eine neutralisierte Reaktionsmischung gemäß Beispiel 1 aufgebracht. Nachdem die Mischung vollständig durchgelaufen war, wurde die obere Hälfte der Säulen entnommen und nach dem Erstarren vermahlen. Man erhielt 880 g Produkt mit einem Gehalt von 31,8 Gew.-% Kokosalkyloligoglucosid und 11.4 Gew.-% Fettalkohol.

## Patentansprüche

1. Verfahren zur Herstellung fester Zuckertenside, **dadurch gekennzeichnet, daß** man Glykosen in an sich bekannter Weise mit Fettalkoholen acetalisiert und die technischen Mischungen, welche neben den Alkyl- und/oder Alkenylglykosiden wenigstens noch Anteile an überschüssigem Fettalkohol enthalten, mit festen Extraktionsmitteln in Kontakt bringt, und dabei eine erste feste Phase, welche das Extraktionsmittel zusammen mit dem ganz überwiegenden Teil der Alkyl- und/oder Alkenyloligoglykosiden und einem gegenüber der Ausgangsmischung verminderten Anteil an Fettalkoholen und eine zweite flüssige Phase erzeugt, welche überwiegend aus abgetrenntem Fettalkohol besteht.

2. Verfahren nach Anspruch 1, **dadurch gekennzeichnet, daß** man feste Zuckertenside herstellt, welche 20 bis 70 Gew.-% Alkyl- und/oder Alkenyloligoglykoside enthalten.

3. Verfahren nach den Ansprüchen 1 und/oder 2, **dadurch gekennzeichnet, daß** man Extraktionsmittel einsetzt, welche gegenüber Alkyl- und/oder Alkenyloligoglykosiden eine höhere Hydrophilie als Fettalkohole aufweisen.

4. Verfahren nach mindestens einem der Ansprüche 1 bis 3, **dadurch gekennzeichnet, daß** man Extraktionsmittel einsetzt, welche wenigstens nach Adsorption der Alkyl- und/oder Alkenyloligoglykosiden bei Raumtemperatur in festem Zustand vorliegen.

5. Verfahren nach mindestens einem der Ansprüche 1 bis 4, **dadurch gekennzeichnet, daß** man Extraktionsmittel einsetzt, welche bei Raumtemparatur fest vorliegen.

6. Verfahren nach mindestens einem der Ansprüche 1 bis 5, **dadurch gekennzeichnet, daß** man Extraktionsmittel einsetzt, welche ausgewählt sind aus der Gruppe, die gebildet wird von Zeolithen, Silicaten, Polymeren, Polysacchariden sowie Salzen anorganischer und/oder organischer Säuren.

7. Verfahren nach mindestens einem der Ansprüche 1 bis 6, **dadurch gekennzeichnet, daß** man die feste Phase nach der Extraktion zerkleinert und auf die gewünschte Teilchengröße vermählt.

## Claims

1. A process for the production of solid sugar surfactants, **characterized in that** glycoses are acetalized with fatty alcohols in known manner and the technical mixtures obtained, which still contain at least some excess fatty alcohol besides the alkyl and/or alkenyl oligoglycosides, are contacted with solid extractants, resulting in the formation of two phases, namely a first solid phase which contains the extractant together with by far the predominant part of the alkyl and/or alkenyl oligoglycosides and a reduced amount of fatty alcohols in relation to the starting mixture and a second liquid phase which consists predominantly of the fatty alcohol removed.

2. A process as claimed in claim 1, **characterized in that** solid sugar surfactants containing 20 to 70% by weight of alkyl and/or alkenyl oligoglycosides are produced.

3. A process as claimed in claims 1 and/or 2, **characterized in that** extractants with a higher hydrophilia than fatty alcohols with respect to alkyl and/or alkenyl oligoglycosides are used.

4. A process as claimed in at least one of claims 1 to 3, **characterized in that** extractants present in solid form at room temperature, at least after adsorption of the alkyl and/or alkenyl oligoglycosides, are used.

5. A process as claimed in at least one of claims 1 to 4, **characterized in that** extractants solid at room temperature are used.

6. A process as claimed in at least one of claims 1 to 5, **characterized in that** extractants selected from the group consisting of zeolites, silicates, polymers, polysaccharides and salts of inorganic and/or organic acids are used.

7. A process as claimed in at least one of claims 1 to 6, **characterized in that**, after the extraction step, the solid phase is size-reduced and ground to the required particle size.

## Revendications

1. Procédé de préparation d'agents tensioactifs dérivés de sucre
**caractérisé en ce qu'**
on acétalise des glycoses d'une manière connue en soi avec des alcools gras et on met en contact les mélanges industriels qui renferment à côté des alkyl - et/ou alkényl-glycosides au moins encore des fractions d'alcool gras en excès, avec des agents d'extraction solides et on produit ainsi une première phase solide qui renferme l'agent d'extraction conjointement à la partie tout à fait prépondérante des alkyl - et/ou alkényl-oligoglycosides et à une fraction diminuée par rapport au mélange de départ en alcools gras, et une deuxième phase liquide qui consiste d'une manière prépondérante en un alcool gras séparé.

2. Procédé selon la revendication 1,
**caractérisé en ce qu'**
on produit des agents tensioactifs dérivés de sucre, qui renferment de 20 à 70 % en poids d'alkyl - et/ou d'alkényl-oligoglycosides.

3. Procédé selon les revendications 1 et/ou 2,
**caractérisé en ce qu'**
on met en oeuvre des agents d'extraction qui possèdent par rapport aux alkyl et/ou alkényl-oligoglycosides, une hydrophilie plus élevée que les alcools gras.

4. Procédé selon au moins une des revendications 1 à 3,
**caractérisé en ce qu'**
on met en oeuvre des agents d'extraction qui se présentent au moins après adsorption des alkyls et/ou alkényl-oligoglycosides à l'état solide à température ambiante.

5. Procédé selon au moins une des revendications 1 à 4,
**caractérisé en ce qu'**
on met en oeuvre des agents d'extraction qui se présentent solides à température ambiante.

6. Procédé selon au moins une des revendications 1 à 5,
**caractérisé en ce qu'**
on met en oeuvre des agents d'extraction choisis dans le groupe formé des zéolithes, des silicates, des polymères, des polysaccharides, ainsi que des sels d'acides inorganiques et/ou organiques.

7. Procédé selon au moins une des revendications 1 à 6,
**caractérisé en ce qu'**
on pulvérise la phase solide après l'extraction et on moud à la taille de particules désirée.
